# EUROPÄISCHE PATENTSCHRIFT

(11) **EP 1 740 241 B1**
(45) Veröffentlichungstag und Bekanntmachung des Hinweises auf die Patenterteilung: **19.12.2007**
(21) Anmeldenummer: 05729362.3
(22) Anmeldetag: 19.04.2005
(51) Int. Cl.: A61M 5/315

(54) **VERABREICHUNGSGERÄT MIT PRIMINGFUNKTION**
ADMINISTRATION DEVICE HAVING PRIMING FUNCTION
DISPOSITIF D'ADMINISTRATION D'UN PRODUIT FLUIDE, A FONCTION D'AMORÇAGE

(30) Priorität: 21.04.2004 DE 102004019325
(43) Veröffentlichungstag der Anmeldung: 10.01.2007
(73) Patentinhaber: TecPharma Licensing AG, 3401 Burgdorf (CH)
(72) Erfinder: KIRCHHOFER, Fritz, CH-3454 Sumiswald (CH)
(86) Internationale Anmeldenummer: PCT/CH2005/000215
(87) Internationale Veröffentlichungsnummer: WO 2005/102419

(56) Entgegenhaltungen:
- EP-A- 0 937 477
- WO-A-02/30495
- WO-A-03/020347
- WO-A-20/04007000

## Beschreibung

Die Erfindung betrifft ein Verabreichungsgerät für die Verabreichung eines fluiden Produktes, vorzugsweise für medizinische, einschließlich tiermedizinische, therapeutische, diagnostische, pharmazeutische und/oder kosmetische Anwendungen. Injektionsgeräte, insbesondere Injektionspens, und auch Inhalationsgeräte sind besonders bevorzugte Beispiele von Verabreichungsgeräten.

In den oben genannten bevorzugten Anwendungen ist es oftmals wichtig, dass eine ganz bestimmte Produktdosis, das heißt eine definierte Produktmenge, verabreicht wird. Eine Quelle der Unsicherheit ist diesbezüglich der Umstand, dass ein Teil des Geräts, der das Produkt, insbesondere medizinisches Produkt oder Medikament, wie zum Beispiel Insulin oder Heparin enthält, nicht gänzlich mit dem Produkt gefüllt ist, sondern Luft enthält. Ohne Entlüftung würde die Luft oder allgemeiner ein Gas, zusammen mit dem Produkt verabreicht werden. Bei beispielsweise subkutaner Verabreichung würde dies zwar nicht zu gesundheitlichen Komplikationen führen, aber immerhin würde nicht die korrekte Produktdosis verabreicht werden, sondern eine um den Luftgehalt verminderte Dosis. Bei anderen Arten der Verabreichung kann es jedoch zusätzlich auch zu gesundheitlichen Beeinträchtigungen kommen, wenn Luft zusammen mit dem Produkt verabreicht wird. Luft kann in dem produktführenden Teil des Verabreichungsgeräts nicht nur dann vorhanden sein, wenn erstmals aus einem Reservoir verabreicht wird, sondern auch noch nach einer ersten Verabreichung wieder oder überhaupt erstmals eintreten. So kann ein flüssiges Produkt in einer Injektionskanüle beispielsweise von der Kanülenspitze her verdunsten und die Kanüle eintrocknen. Falls ein Nachfließen des Produkts von der Seite des Reservoirs her auf Grund der herrschenden Druckverhältnisse nicht oder nicht ausreichend rasch möglich ist, bildet sich in der Injektionskanüle von der Spitze her ein Luftvolumen. Wird später ohne vorherige Entlüftung erneut Produkt verabreicht, so ist die verabreichte Produktdosis diesem Luftvolumen entsprechend vermindert.

Verabreichungsgeräte, wie die Erfindung sie beispielsweise auch betrifft, sind aus der WO 97/36625, der DE 199 00 792 C1 und der US 6,228,067 B1 bekannt. Die dort beschriebenen Verabreichungsgeräte sind Injektionspens, die eine Auswahl der per Injektion zu verabreichenden Produktdosis erlauben. Um die bekannten Geräte zu Primen, wie das Entlüften im Allgemeinen bezeichnet wird, benötigt der Verwender einige Erfahrung und Übung. Der Verwender drückt "nach Gefühl" Produkt durch die Injektionsnadel des jeweiligen Gerätes, bis er durch Sichtkontrolle den Austritt von Produkt an der Nadelspitze feststellt. Hierbei ist es möglich, dass unnötig viel Produkt zum Zwecke des Primens ausgeschüttet wird. Überdies ist eine Sichtkontrolle für Menschen mit beeinträchtigter Sehkraft oftmals schwierig und bewirkt ein erhöhtes Risiko, dass der Primingvorgang nicht korrekt durchgeführt wird.

Aus der WO 2004/007000 A1 ist ein Injektionsgerät mit Primingfunktion bekannt. Hierbei entfällt das Primen nach Gefühl, wobei mittels einer Bewegung quer zur Längsachse des Verabreichungsgerätes zwischen einer Primingfunktion und einer Wirkstofffunktion gewechselt werden kann. Bevorzugt erfolgt dieser Wechsel durch eine Drehbewegung. Diese Bewegung muss exakt ausgeführt werden, um eine entsprechend korrekte Funktion des Verabreichungsgerätes zu gewährleisten. Dies kann bei einer Beeinträchtigung der Feinmotorik des Verwenders Probleme hervorrufen.

Es ist eine Aufgabe der Erfindung, ein Verabreichungsgerät mit Primingfunktion bereitzustellen, das einfach zu bedienen ist. Auf Grund der Bedienerfreundlichkeit wird die Sicherheit dafür erhöht, dass eine bestimmte, dem Verwender bekannte Produktdosis auch tatsächlich verabreicht wird.

Die Bedienerfreundlichkeit kann beispielsweise dadurch gesteigert werden, dass zur Betätigung des Verabreichungsgerätes eine Bewegung auszuführen ist, die dem Verwender bereits aus dem Alltagsgebrauch anderer Gegenstände bekannt ist. Ein solcher allgemein gebräuchlicher Gegenstand ist beispielsweise ein Kugelschreiber. Das Betätigen eines Kugelschreiberdruckknopfes ist einfach auszuführen, und es wird sicher zwischen schreibendem und nichtschreibendem Zustand gewechselt.

Ein Verabreichungsgerät für die Verabreichung eines fluiden Produkts, wie die Erfindung es betrifft, umfasst ein Gehäuse, eine Abtriebsvorrichtung, eine Antriebsvorrichtung, eine Betätigungsvorrichtung und eine Rückstellvorrichtung. Das Gehäuse umfasst ein Reservoir für das fluide Produkt. Der Begriff Fluid betrifft hierbei sowohl Gase als auch Flüssigkeiten. Die Abtriebsvorrichtung wirkt auf das im Reservoir enthaltene Produkt, um das Produkt auszuschütten. Der Begriff Ausschüttung ist hierbei allgemein aufzufassen, er kann sich sowohl auf das Ausschütten des fluiden Produktes zum Priming als auch auf die Ausschüttung des fluiden Produktes zum gezielten Wirkstoffeinsatz beziehen. Die Antriebsvorrichtung wirkt auf die Abtriebsvorrichtung ein. Sie stellt damit sicher, dass die Abtriebsvorrichtung, die auf das im Reservoir enthaltene Produkt wirkt, das Produkt ausschüttet. Die Antriebsvorrichtung sorgt für die Energieübertragung auf die Abtriebsvorrichtung. Der Antrieb selber kann hierbei beispielsweise mechanisch oder elektrisch erfolgen. Die Betätigungsvorrichtung ist beweglich und mit der Antriebsvorrichtung gekoppelt. Durch die Betätigung der Betätigungsvorrichtung bevorzugt nach dem Druckknopfprinzip wird somit der Mechanismus der Antriebsvorrichtung in Gang gesetzt. Die Betätigungsvorrichtung ist beweglich, sie kann beispielsweise verschoben, zusammengedrückt oder gedreht werden. Die Betätigungsvorrichtung kann einteilig oder mehrteilig ausgebildet sein. Im Falle der Mehrteiligkeit können die einzelnen Bestandteile aus demselben Material oder aus verschiedenen Materialien hergestellt sein. Die Kopplung der Betätigungsvorrichtung mit der Antriebsvorrichtung ist bevorzugt eine mechanische, es sind aber auch andere Kopplungsarten denkbar. Die Betätigungsvorrichtung weist weiterhin wenigstens zwei unterschiedliche Ausgangsstellungen auf, ausgehend von denen die Betätigungsvorrichtung zur Ausschüttung des fluiden Produktes in Bewegung gesetzt wird. Diese unterschiedlichen Ausgangsstellungen können durch unterschiedliche Ausgangspositionen definiert sein. Beispielsweise befinden sich zwei Ausgangsstellungen bezüglich der zentralen Längsachse des Verabreichungsgerätes auf unterschiedlichen Seiten, sie können auch bezüglich ihrer Position projiziert auf die Längsachse unterschiedlich weit von den Enden des Verabreichungsgerätes, wie z.B. einer Injektionsnadel, entfernt sein. Die unterschiedlichen Ausgangsstellungen können auch auf Grund einer Verformung der Betätigungsvorrichtung definiert sein. Diese können beispielsweise durch ein Zusammendrücken der Betätigungsvorrichtung resultieren. Bevorzugt werden die unterschiedlichen Ausgangsstellungen der Betätigungsvorrichtung durch eine jeweils unterschiedliche Orientierung eines Teiles der Betätigungsvorrichtung bezüglich der Längsachse des Verabreichungsgerätes definiert. Diese Orientierung wird z.B. durch eine Drehung oder Kippen eines Bestandteiles der Betätigungsvorrichtung oder durch eine Drehung oder Kippen in Kombination mit einer Verschiebung erreicht. Die Rückstellvorrichtung des Verabreichungsgerätes bewegt bevorzugt selbsttätig bzw. automatisch zum Beispiel durch Einsatz eines elastischen Mittels (zum Beispiel Feder oder Gummi) die Betätigungsvorrichtung nach der Bewegung aus der einen Ausgangsstellung heraus zurück in die andere bzw. eine weitere Ausgangsstellung. Die hierbei auftretende Rückstellbewegung der Betätigungsvorrichtung kann eine Dreh- oder Kippbewegung und/oder eine translatorische Bewegung beinhalten. Das Verabreichungsgerät ist so ausgebildet, dass der Bewegung der Betätigungsvorrichtung aus den verschiedenen Ausgangsstellungen heraus unterschiedliche Mengen an ausgeschüttetem Fluid zugeordnet sind. Die Bewegung der Betätigungsvorrichtung aus den verschiedenen Ausgangsstellungen heraus initiiert somit die Ausschüttung des Fluides, die jeweilige Ausgangsstellung definiert die auszuschüttende Menge des Produktes. Die Ausschüttung des Fluides kann gleichzeitig mit dem Bewegungsbeginn erfolgen, die Ausschüttung kann aber auch zeitlich verzögert beginnen. In einer bevorzugten Ausführungsform des Verabreichungsgerätes erfolgt die Produktausschüttung gleichzeitig mit der Bewegung der Betätigungsvorrichtung und endet in dem Moment, in dem die Bewegung der Betätigungsvorrichtung endet.

Im Falle der Ausbildung von zwei Ausgangsstellungen ist es bevorzugt so, dass es sich bei der einen Menge des ausgeschütteten fluiden Produktes um eine Primingmenge handelt, bei der anderen Menge des ausgeschütteten fluiden Produktes handelt es sich um eine Wirkstoffmenge, die zum dosierten Wirkstoffeinsatz benutzt wird. Hierbei ist vorteilhafterweise die Primingmenge deutlich kleiner als die Wirkstoffmenge, um das Produkt nicht zu verschwenden.

Bevorzugt besteht das Betätigen der Betätigungsvorrichtung aus einer Bewegung eines Elementes der Betätigungsvorrichtung entlang der Längsachse des Verabreichungsgerätes. Dieses Betätigen kann beispielsweise im Drücken eines Druckknopfes in Richtung besagter Längsachse bestehen. Prinzipiell ist aber auch eine Drehbewegung zum Betätigen der Betätigungsvorrichtung möglich oder eine Kombination aus einer translatorischen Bewegung und einer Drehbewegung.

Gemäß einer bevorzugten Ausführungsform der Erfindung umfasst die Rückstellvorrichtung ein elastisches Element. Dieses elastische Element kann z.B. verformt werden, bevorzugt handelt es sich um eine Federung. Es ist prinzipiell aber ebenfalls denkbar, anstelle des elastischen Elementes ein unelastisches Element zu verwenden, das zwecks der Rückstellung mechanisch oder elektrisch angetrieben bewegt wird.

Gemäß einer weiteren bevorzugten Ausführungsform der Erfindung endet die Rückwärtsbewegung der Betätigungsvorrichtung in zwei verschiedenen Ausgangsstellungen. Unter einer Rückwärtsbewegung wird eine der ursprünglichen Bewegung entgegengesetzt gerichtete Bewegung verstanden. Enthält beispielsweise die Bewegung der Betätigungsvorrichtung während der Produktausschüttung einen Bewegungsanteil in Richtung der Längsachse des Verabreichungsgerätes hin zur Produktaustrittsöffnung, so liegt bei der Rückwärtsbewegung ein Bewegungsanteil entlang der Längsachse des Verabreichungsgerätes weg von der Produktaustrittsöffnung vor. Diese rein translatorischen Bewegungen können natürlich auch von Dreh- oder Kippbewegungen überlagert sein. Diese Dreh- oder Kippbewegungen können während der Hinbewegung und während der Rückwärtsbewegung der Betätigungsvorrichtung deckungsgleich verlaufen, bevorzugt jedoch unterscheiden sie sich.

Bevorzugt weist das Verabreichungsgerät eine Endstellung auf, durch die die Bewegung der Betätigungsvorrichtung zur Beendigung der Ausschüttung des fluiden Produktes begrenzt wird. Hierbei beginnt also die Produktausschüttung der unterschiedlichen Mengen in verschiedenen Ausgangsstellungen, die Endstellung zur Beendigung der Ausschüttung ist jedoch in beiden Fällen dieselbe. Befinden sich beispielsweise zwei Ausgangsstellungen bezüglich der zentralen Längsachse des Verabreichungsgerätes auf unterschiedlichen Seiten und sind ihre Positionen projiziert auf die Längsachse unterschiedlich weit von den Enden des Verabreichungsgerätes entfernt und wird nun die Bewegung der Betätigungsvorrichtung aus den verschiedenen Ausgangsstellungen heraus direkt und kontinuierlich in eine Produktausschüttung bis zu einer gemeinsamen Endstellung umgesetzt, so ist unmittelbar ersichtlich, dass durch die gemeinsame Endstellung unterschiedliche Produktausschüttungsmengen resultieren. Prinzipiell sind jedoch auch mehrere Endstellungen denkbar, die den jeweiligen Ausgangspositionen zugeordnet sind und die die jeweilige Produktmenge festlegen, die ausgeschüttet wird. Bevorzugt wird jedoch eine einzige Endstellung verwendet, was die Herstellung des Verabreichungsgerätes vereinfacht.

Bevorzugt wird oben genannte Endstellung durch einen Anschlag gebildet. Bevorzugt wird hierbei ein beweglicher Teil der Betätigungsvorrichtung bei Berühren des Anschlags an seiner weiteren Bewegung gehindert.

Bevorzugt erfolgt die Kopplung der Betätigungsvorrichtung mit der Antriebsvorrichtung durch ein Kopplungsstück. Dieses Kopplungsstück kann einteilig oder mehrteilig ausgebildet sein. Das Kopplungsstück ist beweglich, es kann in eine oder mehrere Richtungen verschiebbar und/oder drehbar sein. Das Kopplungsstück kann starr oder elastisch ausgebildet sein.

In einer bevorzugten Ausführungsform umfasst das Verabreichungsgerät zwei Anschläge, die mit dem Kopplungsstück wechselweise in Verbindung gebracht werden. Bevorzugt definiert die Stellung des Kopplungsstückes bezüglich der beiden Anschläge die beiden Ausgangsstellungen einer erfindungsgemäßen Vorrichtung. Bei der Verbindung des Kopplungsstückes mit einem Anschlag kann es sich um eine bloße Berührung handeln, bevorzugt sind die zwei Anschläge jedoch als Rastanschläge ausgebildet, die mit dem Kopplungsstück bevorzugt mit Spiel verrasten bzw. verhaken. Prinzipiell sind auch andere Formen einer Verbindung möglich, beispielsweise eine magnetische Fixierung. Unter einer wechselweisen Verbindung wird eine Verbindung verstanden, die abwechselnd mal mit dem einen Anschlag und mal mit dem anderen Anschlag in Verbindung ist. Vorteilhafterweise ist das Kopplungsstück niemals mit beiden Rastanschlägen gleichzeitig verbunden, prinzipiell könnte dies jedoch übergangsweise zwischen der Verbindung des Kopplungsstückes mit dem einen Rastanschlag und der Verbindung des Kopplungsstückes mit dem anderen Rastanschlag der Fall sein.

In einer bevorzugten Ausführungsform der Erfindung ist einer der oben genannten Anschläge ein Priminganschlag und der andere ist ein Wirkstoffanschlag. Ausgehend vom Priminganschlag wird ein Priming eingeleitet und ausgehend vom Wirkstoffanschlag wird durch eine Bewegung der Betätigungsvorrichtung eine definierte Wirkstoffverabreichung ausgelöst.

Bevorzugt besteht das Kopplungsstück aus einem Übertragungsstück und einem Schiebestück. Diese sind miteinander verbunden. Sowohl das Übertragungsstück als auch das Schiebestück können einteilig oder mehrteilig ausgebildet sein. Bevorzugt sind das Übertragungsstück und das Schiebestück miteinander im Eingriff. Bevorzugt besteht dabei ein Spiel zwischen Übertragungsstück und Schiebestück. Hierzu weist bevorzugt das Übertragungsstück eine Aussparung auf, in die das Schiebestück eingreift.

Bevorzugt ist das Übertragungsstück zweischenklig ausgebildet. Unter einer zweischenkligen Ausbildung können beispielsweise V-förmige oder U-förmige Übertragungsstücke verstanden werden. Die zwei Schenkel des Übertragungsstückes können unterschiedlich groß oder gleich groß ausgebildet sein, sie können zueinander eine Symmetrie aufweisen oder gänzlich unterschiedlich geformt sein. Im Sinne der Erfindung kann Zweischenkligkeit auch durch die Funktionalität der Schenkel definiert werden, beispielsweise gerät der eine Schenkel in Kontakt mit einem Priminganschlag, der andere mit einem Wirkstoffanschlag.

Ist das erfindungsgemäße Verabreichungsgerät mit einem Übertragungsstück und mit zwei Anschlägen bevorzugt Rastanschlägen versehen, so weist das Übertragungsstück vorzugsweise zwei gewinkelte Abschnitte auf, die mit den zwei Anschlägen verhaken. Insbesondere können bei einem zweischenkligen Übertragungsstück die beiden Schenkel jeweils einen gewinkelten Abschnitt aufweisen. Die gewinkelten kleinen Abschnitte dienen zum besseren Verhaken des Übertragungsstückes mit den zwei Anschlägen.

In einer bevorzugten Ausführungsform der Erfindung ist das Übertragungsstück kippbar und/oder drehbar um die Rastanschläge. Unter einer Kippbewegung wird eine rotatorische Bewegung um einen Drehpunkt verstanden. Dieser Bewegung kann gegebenenfalls eine translatorische Bewegung überlagert sein. Die Kipp- oder Drehbewegung des Übertragungsstückes tritt bei der vorliegenden Erfindung bevorzugt in folgender Situation auf: Ein Schenkel des Übertragungsstückes ist an seinem Ende leicht gewinkelt und mit diesem gewinkelten Ende an einem Rastanschlag verhakt. Wird das Übertragungsstück nun durch ein Betätigen der Betätigungsvorrichtung in Bewegung versetzt, so kann es dadurch beispielsweise unter Druck oder Zug geraten. Es bleibt dabei zunächst mit den Rastanschlägen verbunden und weicht durch Kippen oder Drehen der einwirkenden Kraft aus. Anschließend löst sich das Übertragungsstück von dem einen Rastanschlag und wird in einer Rückwärtsbewegung zum zweiten Rastanschlag bewegt, mit dem sich der andere Schenkel verhakt.

In einer bevorzugten Ausführungsform ist das Schiebestück des Verabreichungsgerätes in einer Richtung orthogonal zur Längsachse des Verabreichungsgerätes verschiebbar. Diese Bewegung kann mit einer Bewegung in Richtung der Längsachse des Verabreichungsgerätes super positioniert sein. Bevorzugt ist das Schiebestück orthogonal zur Längsachse des Verabreichungsgerätes von einer Gehäusewandung des Verabreichungsgerätes zur bezüglich der Längsachse des Verabreichungsgerätes gegenüber liegenden Gehäusewandung verschiebbar. Bevorzugt gerät das Schiebestück bei Kontakt mit den Gehäuseswandungen dort in Passform zum Anliegen. In einer bevorzugten Ausführungsform wird die Bewegung des Schiebestückes in Richtung der Längsachse des Verabreichungsgerätes direkt auf die Bewegung der Antriebsvorrichtung bzw. der Abtriebsvorrichtung des Verabreichungsgerätes und damit auf die Ausschüttung des Produktes übertragen.

In einer bevorzugten Ausführungsform weist das Verabreichungsgerät ein zweischenkliges symmetrisches Übertragungsstück auf, das mit einem verschiebbaren Schiebestück mit Spiel in Eingriff ist. Die Symmetrieachse des Übertragungsstücks ist in einer ersten Ausgangsstellung gegenüber der Längsachse des Verabreichungsgerätes verkippt und verhakt mit einem Schenkel an einem ersten Rastanschlag. Das Betätigen der Betätigungseinrichtung erfolgt durch Drücken eines Knopfes und Kraftübertragung dieser Druckkraft auf das Übertragungsstück entlang der Längsachse des Verabreichungsgerätes. Aufgrund der bezüglich der Längsachse des Verabreichungsgerätes verkippten Lage der Symmetrieachse des Übertragungsstückes und seines Eingriffes in das Schiebestück kommt es zu einer Hebelwirkung des Übertragungsstückes auf das Schiebestück. Das Übertragungsstück löst sich unter zunehmendem Druck der Betätigungsvorrichtung vom ersten Rastanschlag und bewegt das Schiebestück in Richtung auf den zweiten Rastanschlag entlang der Querachse des Verabreichungsgerätes. Nach dem Loslassen der Betätigungsvorrichtung bewegt die Rückstellvorrichtung das Übertragungsstück zwangsläufig in die zweite Ausgangsstellung, das heißt bei seiner Rückwärtsbewegung verhakt das Übertragungsstück nun automatisch mit seinem zweiten Schenkel an einem zweiten Rastanschlag. Durch dieses Verhaken und die entlang der Längsachse des Verabreichüngsgerätes wirkende Rückstellkraft ändert sich die Orientierung der Symmetrieachse des Übertragungsstückes bezüglich der Längsachse des Verabreichungsgerätes, die Symmetrieachse ist in der zweiten Ausgangsstellung in die andere Richtung bezüglich der Längsachse verkippt als in der ersten Ausgangsstellung. Die Vorgänge bei der Betätigung des Verabreichungsgerätes aus der zweiten Ausgangsstellung heraus laufen analog ab und enden automatisch in der ersten Ausgangsstellung.

In einer bevorzugten Ausführungsform der Erfindung weist das Verabreichungsgerät ein Dosierglied auf, mit dem die zu verabreichende Wirkstoffmenge des fluiden Produktes eingestellt wird. Es ist also beispielsweise möglich, eine konstant bleibende geringe Primingmenge mit einer einstellbaren, insbesondere wechselnden, tatsächlich zu verabreichenden Wirkstoffmenge des fluiden Produktes zu kombinieren.

Gemäß einer bevorzugten Ausführungsform weist das Verabreichungsgerät eine Abtriebsvorrichtung mit einem Kolben auf, der bei Produktverabreichung in eine Richtung entlang der Längsachse des Verabreichungsgerätes bewegt wird und der an einer Bewegung in die entgegengesetzte Richtung durch eine Sperrvorrichtung gehindert wird. Dies bietet eine zusätzliche Sicherheit dafür, dass ein ungewollter Lufteintritt in das Verabreichungsgerät verhindert wird. Die Sperrvorrichtung kann beispielsweise aus einer Zahnreihe bestehen, entlang dieser Zahnreihe ist eine Bewegung nur in eine Richtung entlang der Zahnreihe möglich. Die Sperrvorrichtung kann alternativ aus einem speziell gebogenen Kunststoff gefertigt sein, der ein Zurückrutschen des Kolbens verhindert.

Gemäß einem weiteren Aspekt bezieht sich die Erfindung auf ein Verfahren zur Verwendung des Verabreichungsgerätes. Dieses Verfahren umfasst die folgenden Schritte: erstes Betätigen der Betätigungsvorrichtung in einer ersten Ausgangsstellung, Ausschütten einer ersten Menge eines fluiden Produktes, Rückstellen der Betätigungsvorrichtung in eine zweite Ausgangsstellung, zweites Betätigen der Betätigungsvorrichtung in der zweiten Ausgangsstellung, Ausschütten einer zweiten Menge eines fluiden Produktes, Rückstellen der Betätigungsvorrichtung in die erste Ausgangsstellung. Das Verfahren beginnt also in einer ersten Ausgangsstellung, in der sich die Betätigungsvorrichtung bzw. wenigstens ein Bestandteil der Betätigungsvorrichtung befindet. Das Betätigen der Betätigungsvorrichtung löst zeitgleich oder verzögert die Ausschüttung einer ersten Menge eines fluiden Produktes aus. Bevorzugt besteht das Betätigen der Betätigungsvorrichtung in dem Drücken eines Betätigungsknopfes, wie es den Verwendern von Verabreichungsgeräten bereits von der Benutzung von Kugelschreibern her bekannt ist. Der Druckknopf lässt sich bis zu einem gewissen Grad eindrücken, dann stößt er auf eine Barriere, nach Loslassen des Druckknopfes erfährt der Druckknopf eine Rückstellbewegung. Das Rückstellen der Betätigungsvorrichtung erfolgt in eine zweite Ausgangsstellung. Diese zweite Ausgangsstellung kann sich von der ersten Ausgangsstellung beispielsweise dadurch unterscheiden, dass ihre Positionen unterschiedlich weit von der Produktaustrittsöffnung des Verabreichungsgerätes entlang der Längsachse des Verabreichungsgerätes entfernt sind oder die Betätigungsvorrichtung unterschiedliche Positionen relativ zum Gehäuse einnimmt. Eben solche Verhältnisse sind bei dem Beispiel eines herkömmlichen Kugelschreibers anzutreffen. Abhängig davon, ob mit dem Kugelschreiber geschrieben werden kann oder nicht, befindet sich der Druckknopf des Kugelschreibers unterschiedlich weit entfernt von der Kugelschreiberspitze bzw. ragt die Schreibspitze des Kugelschreibers aus diesem heraus oder ist in ihm verborgen. In einer anderen Ausführungsform unterscheiden sich die beiden Ausgangsstellungen beispielsweise auch durch einen unterschiedlichen Drehwinkel bezüglich der Längsachse des Verabreichungsgerätes. Das zweite Betätigen der Betätigungsvorrichtung unterscheidet sich vom ersten Betätigen der Betätigungsvorrichtung dadurch, dass es in der zweiten Ausgangsstellung ausgelöst wird. Es initiiert einen Prozess zur Ausschüttung einer zweiten Menge eines fluiden Produktes. Diese Ausschüttung kann zeitgleich mit der Betätigung der Betätigungsvorrichtung erfolgen oder auch zeitlich verzögert sein. Das erneute Rückstellen der Betätigungsvorrichtung erfolgt nach der Ausschüttung einer zweiten Menge eines fluiden Produktes in die erste Ausgangsstellung. Bevorzugt kommt bei der vorliegenden Erfindung eine Kugelschreibermechanik zum Einsatz. Bei der Betätigung in der ersten Ausgangsstellung wird das Ausschütten der ersten Menge erzielt und bei der Betätigung in der zweiten Ausgangsstellung wird die zweite Menge ausgeschüttet.

Das beschriebene Verfahren wird zyklisch durchlaufen, es kann dementsprechend beliebig oft wiederholt werden. Das Verfahren insgesamt kann ebenfalls analog zur Betätigung eines Kugelschreibers verstanden werden. Bei einem Kugelschreiber ist es auch möglich, durch mehrfaches Betätigen des Kugelschreiber-Druckknopfes zwischen schreibfähigem und nicht schreibfähigem Zustand des Kugelschreibers zu wechseln.

Ein Ausführungsbeispiel der Erfindung wird nachfolgend anhand von Figuren beschrieben. Mit der Offenbarung dieses Ausführungsbeispieles ist keine Einschränkung der Erfindung bezweckt. Es handelt sich lediglich um eine von mehreren Möglichkeiten, ein erfindungsgemäßes Verabreichungsgerät zu konstruieren. Es zeigen:
- Fig. 1: ein Injektionsgerät in einem Längsschnitt,
- Fig. 2A bis 2E: den Betätigungsabschnitt.

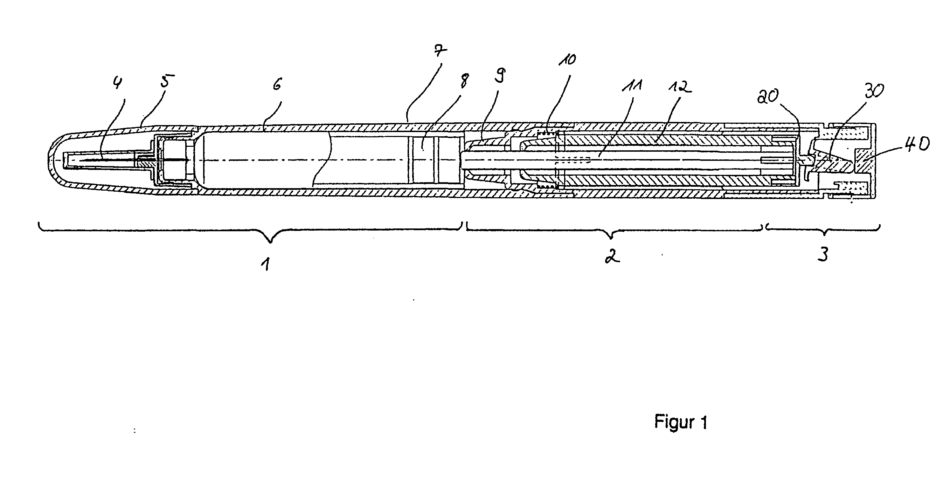

Fig. 1 zeigt ein Injektionsgerät in einem Längsschnitt. Im gezeigten Beispiel lässt sich das Injektionsgerät in drei Hauptabschnitte unterteilen: einen Injektionsabschnitt 1, einen Antriebsabschnitt 2 und einen Betätigungsabschnitt 3. Diese Unterteilung ist nicht strikt aufzufassen, sie ist nur eine von mehreren Möglichkeiten.

Der Injektionsabschnitt 1 enthält in einem Gehäuse 7 ein Reservoir 6, in dem sich das fluide Produkt befindet. Aus dem Reservoir 6 gelangt das fluide Produkt in eine Injektionsnadel 4, mit der der Wirkstoff beispielsweise in einen menschlichen Körper injiziert wird. Wird das Injektionsgerät gerade nicht benutzt, so wird die Nadel 4 durch eine Schutzkappe 5 geschützt.

Mit Hilfe eines Kolbens 8 wird das fluide Produkt aus dem Injektionsgerät herausgedrückt. Im vorliegenden Beispiel wird der Kolben 8 mittels einer Kolbenstange 11 bewegt. Kolben 8 und Kolbenstange 11 können nur in Richtung der Austrittsöffnung des Injektionsgerätes bewegt werden, hierfür sorgt eine Sperrvorrichtung 9.

Der Betätigungsabschnitt 3 enthält wesentliche Bestandteile der Betätigungsvorrichtung. Im vorliegenden Fall sind dies der Betätigungsknopf 40 der Betätigungsvorrichtung, ein V-förmiges Übertragungsstück 30 und ein verschiebbares Schiebeteil 20. Übertragungsstück 30 und Schiebeteil 20 bilden zusammen ein Kopplungsstück. Außerhalb des Betätigungsabschnitts 3 im Antriebsabschnitt 2 befindet sich eine Federung 10, die funktionell ebenfalls der Betätigungsvorrichtung zuzurechen ist. Durch ein Eindrücken des Betätigungsknopfes 40 wird das Übertragungsstück 30 verschoben und gedreht. Dieses Übertragungsstück 30 ist bevorzugt mit Spiel mit dem Schiebeteil 20 in Eingriff, was sich entsprechend der Bewegung des Übertragungsstückes 30 mitbewegt. Das Schiebeteil 20 wirkt auf das Mitnehmerteil 12, das wiederum, bei Bewegung auf die Austrittsöffnung zu, die Antriebsvorrichtung bzw. die Kolbenstange 11 mitnimmt und somit den Kolben 8 in Richtung der Austrittsöffnung des Injektionsgerätes bewegt, so dass Produkt aus dem Injektionsgerät herausgedrückt wird. Bewegt sich das Schiebeteil 20 nach der Produktausschüttung wieder in rückwärtiger Richtung weg von der Austrittsöffnung des Injektionsgerätes aufgrund der Kraft der Federung 10, so folgt die Kolbenstange 11 dieser Bewegung nicht. Die Kolbenstange 11 wird hieran durch die Sperrvorrichtung 9 gehindert. Das Mitnehmerteil 12 folgt aber dieser Bewegung und nimmt rastend eine neue Relativstellung zur Kolbenstange 11 ein. Während wiederholter Produktausschüttungsvorgänge entfernt sich die Kolbenstange 11 also immer weiter vom Schiebestück 20 bzw. vom Ende des Injektionsgerätes, an dem sich die Betätigungsvorrichtung 3 befindet, während das Mitnehmerteil 12 damit in Kontakt bleibt.

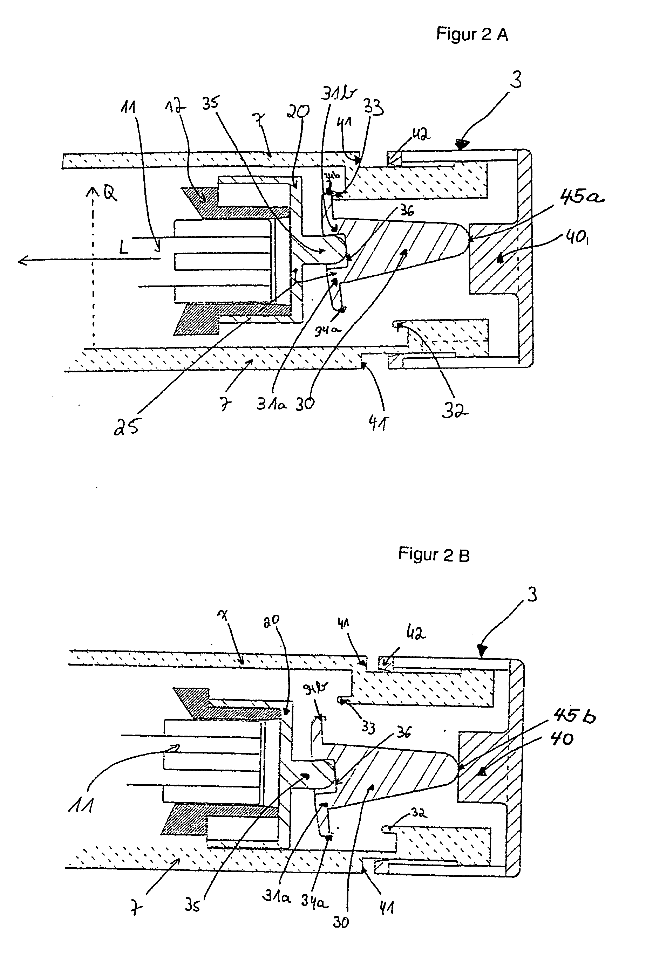

Die Fig. 2A bis 2E zeigen in detaillierterer Darstellung den Betätigungsabschnitt 3 in vergrößerter Form. In den Fig. 2A bis 2E soll exemplarisch die Funktionsweise einer erfindungsgemäßen Betätigungsvorrichtung verdeutlicht werden.

Fig. 2A zeigt den Betätigungsabschnitt 3, direkt bevor das Primen des Injektionsgerätes erfolgt. Im Wesentlichen ist der Betätigungsabschnitt 3 von einem Gehäuse 7 eingefasst. Der hintere Teil wird jedoch direkt durch einen Betätigungsknopf 40 gebildet. Dieser weist eine kappenhafte Ausführungsform auf und ist auf das Gehäuse 7 aufgesteckt. Der Betätigungsknopf 40 ist bezüglich des Gehäuses 7 entlang der Längsachse L verschiebbar bis in eine Endstellung 41, die als Anschlag ausgebildet ist. Der bezüglich der Längsachse L zentrale Bereich des Betätigungsknopfes ist als Erhebung ausgebildet. Auf dieser Erhebung liegt das Übertragungsstück 30 in einer Position 45a an. Das Übertragungsstück 30 ist in etwa V-förmig geformt und ist bezüglich der Längsachse L leicht verkippt. Es weist eine Aussparung 36 auf und es untergliedert sich in zwei, in diesem Fall symmetrische Schenkel 31a und 31b. Am Ende dieser Schenkel 31a und 31b befinden sich jeweils kleine gewinkelte Abschnitte 34a und 34b, diese gewinkelten Abschnitte 34a und 34b können die Funktionsweise eines Häkchens wahrnehmen. Der eine Schenkel 31 b des Übertragungsstückes 30 ist mit einem Rastanschlag 33 in Kontakt, dieser ist aus demselben Material und Werkstück wie das Gehäuse 7 selbst gefertigt. Der Rastanschlag 33 ist in einem gewissen Abstand von der Wandung des Injektionsgerätes längs bezüglich der Längsachse L ausgebildet. Das Verhaken des Schenkels 31b mittels des Häkchens 34b an dem Rastanschlag 33 erfolgt vorzugsweise mit einem gewissen Spiel.

Das Häkchen 34b kann sich um den Rastanschlag 33 herum drehend bewegen bzw. kippen. Die andere Seite des Übertragungsstückes 30 mit dem anderen Schenkel 31a und dem Häkchen 34a befindet sich vor dem Beginn des Primingvorganges bevorzugt in keinerlei Kontakt.

In die Aussparung 36 greift ein Vorsprung 35 eines Schiebestückes 20 mit Spiel ein. Das Schiebestück kann entlang der Achse L und orthogonal hierzu entlag der Achse Q bewegt werden. Das Schiebestück 20 ist mit der Kolbenstange 11 in mittelbarem Kontakt, die Kolbenstange 11 kann ausschließlich in Richtung der Achse L bewegt werden. Das Schiebestück 20 ist vor Beginn des Primingvorganges leicht gegenüber der zentralen L-Achse in Richtung der Q-Achse nach oben verschoben. Entsprechend liegt der Fortsatz 35 des Schiebestückes 20 im oberen Bereich des Übertragungsstückes 30 an.

Wird nun der Primingvorgang durchgeführt, so wird der Betätigungsknopf 40 entlang der Achse L gegen die Federkraft der Rückstellvorrichtung 10 eingedrückt, bis die seitlichen Einfassungen 42 des Betätigungsknopfes 40 den Anschlag 41 erreichen. Durch die Bewegung des Betätigungsknopfes wird auch das Übertragungsstück 30 bewegt. Es löst sich mit einer drehenden Bewegung von dem Rastanschlag 33 ab. Das Übertragungsstück 30 wird hierbei also gekippt bzw. verdreht. Es wirkt als Hebel auf das Schiebestück 20 ein.

Fig. 2B zeigt den Betätigungsabschnitt 3 kurz nach der Durchführung des Primingprozesses. Der Betätigungsknopf 40 wurde gerade wieder entlastet und durch die Rückstellvorrichtung 10 wird der Betätigungsknopf 40, vermittelt durch Schiebestück 20 und Übertragungsstück 30, wieder nach hinten bewegt. Es ist bereits wieder eine kleine Lücke zwischen dem Endanschlag 41 und der seitlichen Einfassung 42 des Betätigungsknopfes 40 entstanden. Der Auflagepunkt 45b des Übertragungsstückes 30 hat sich auf dem Betätigungsknopf 40 nach unten verlagert. Das Schiebestück 20 hat sich entlang der Q-Achse nach unten bewegt und liegt nun unten auf dem Gehäuse 7 auf. Entsprechend hat sich auch das Übertragungsstück 30 nach unten bewegt. Bevorzugt ist keiner der beiden Schenkel 31a und 31b des Übertragungsstückes 30 mit seinen gewinkelten Enden 34a und 34b zu diesem Zeitpunkt mit einem Rastanschlag 32 oder 33 in Kontakt.

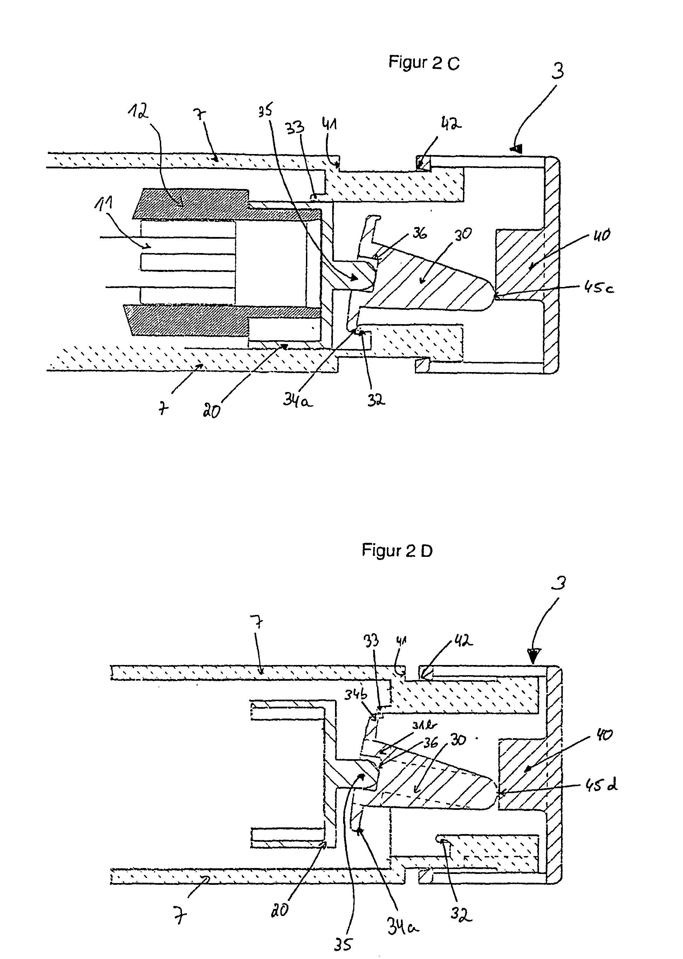

Fig. 2C zeigt den Betätigungsabschnitt 3 zu einem etwas späteren Zeitpunkt, nachdem die Rückstellbewegung durch ein Einhaken des Übertragungsstückes 30 bzw. seines Häkchens 34a mit dem Rastanschlag 32 beendet worden ist. Die Federung 10 bzw. das Schiebestück 20 drückt das Übertragungsstück 30 soweit wie möglich nach hinten. Dadurch wird das Schiebestück 30 bezüglich der Längsachse L des Injektionsgerätes in die entgegengesetzte Richtung wie in Fig. 2A verkippt. Der Auflagepunkt 45c des Übertragungsstückes 30 liegt nun im unteren Bereich der Erhebung des Betätigungsknopfes 40. Das Schiebestück 20 ist im unteren Teil der Aussparung 36 des Übertragungsstückes 30 mit dem Übertragungsstück 30 mit Spiel im Eingriff. Der obere Bereich des Schiebestückes 20 ist in der Position der Fig. 2C unter den Rastanschlag 33 gerutscht bzw. zurückgezogen. Das in Fig. 2C gezeigte Injektionsgerät ist in dieser Position bereit zur Ausschüttung der zu injizierenden Wirkstoffmenge. Der Führungspunkt 42 des Betätigungsknopfes 40 ist in dieser Stellung am weitesten von dem Endanschlag 41 entfernt. Hierdurch kann eine große Wirkstoffmenge im Folgenden ausgeschüttet werden.

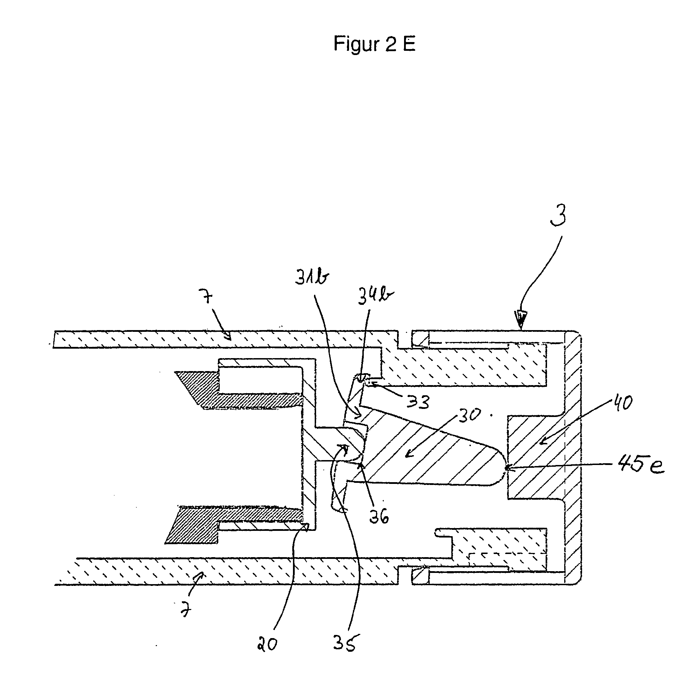

Fig. 2D zeigt das Verabreichungsgerät kurz vor Beendigung des Ausschüttprozesses der zu injizierenden Wirkstoffmenge. Der Führungspunkt 42 des Betätigungsknopfes 40 hat sich bereits ein gutes Stück auf den Endanschlag 41 zu bewegt. Das Häkchen 34a des Verbindungsstückes 30 hat sich bereits wieder von dem hinteren Rastanschlag 32 gelöst. Das Übertragungsstück 30 beginnt sich erneut zu drehen und sich nach vorne in Richtung der L-Achse zu bewegen, der Auflagepunkt 45d auf dem Betätigungsknopf 40 wandert wieder nach oben. Das Schiebestück 20 folgt hierbei passiv dem Übertragungsstück 30. Kurz vor Beendigung der Produktausschüttung gerät das Übertragungsstück 30 mit dem gewinkelten Teil 34b unterhalb des Rastanschlages 33 mit dem Rastanschlag 33 in Kontakt und gleitet an diesem entlang. Ist der Betätigungsknopf 40 vollkommen eingedrückt, so gleitet das Häkchen 34b an dem Rastanschlag 33 vorbei und kann sich bei der folgenden Rückwärtsbewegung daran verhaken. Aus Gründen der Einfachheit wurde auf die Darstellung der Kolbenstange 11 in Fig. 2D sowie auch in der im Folgenden beschriebenen Figur 2E verzichtet.

Fig. 2E zeigt das Injektionsgerät kurz vor der Dosierung der Primingmenge. Der Schenkel 31b des Übertragungsstückes 30 hat sich bereits mit seinem gewinkelten Fortsatz 34b um den Primingrastanschlag 33 verhakt. Im weiteren Verlauf dieser zweiten Rückstellbewegung wird der Betätigungsknopf 40 noch geringfügig nach rechts, also von der Öffnung des Injektionsgerätes weg, bewegt. Auf Grund des Verhakens des Übertragungsstückes 30 mit dem Primingrastanschlag 33 erfährt das Übertragungsstück 30 bei dieser Bewegung eine Drehung um den Rastanschlag 33 herum, das Übertragungsstück 30 wird insgesamt noch etwas weiter verdreht bzw. gekippt. Bei dieser Bewegung wandert die Symmetrieachse des Übertragungsstückes 30 über die zentrale Längsachse L des Injektionsgerätes hinweg und ist abschließend, wie bereits in Fig. 2A gezeigt, bezüglich der Steigung der Symmetrieachse anders zur Längsachse L orientiert.

Der in den Fig. 2A bis 2E beschriebene Prozess kann zyklisch und entsprechend mehrfach durchlaufen werden. Über die bei dem jeweiligen Prozessschritt auszuschüttende Produktmenge entscheidet die jeweilige Rastposition 32 bzw. 33, aus denen heraus der Ausschüttvorgang begonnen wird. Entsprechend der eingenommenen Rastpositionen 32 und 33 ist auch der von dem Betätigungsknopf 40 zurück zu legende Weg zwischen dem Führungspunkt 42 bis hin zu dem gemeinsamen Endanschlag 41 unterschiedlich lang. Diese zwei verschiedenen Längen stehen in direktem Verhältnis zu den zwei unterschiedlichen Produktmengen, die im einen Fall als Primingmenge und im anderen Fall als Wirkstoffmenge ausgeschüttet werden.

## Patentansprüche

1. Verabreichungsgerät für die Verabreichung eines fluiden Produktes, umfassend:
a) ein Gehäuse (7) mit einem Reservoir (6) für das Produkt;
b) eine Abtriebsvorrichtung (8), die ausgebildet ist, das Produkt auszuschütten, wenn es sich im Reservoir befindet;
c) eine auf die Abtriebsvorrichtung (8) wirkende Antriebsvorrichtung (11);
d) eine Betätigungsvorrichtung (3),
- die beweglich ist,
- die mit der Antriebsvorrichtung (11) gekoppelt ist, und
- die mindestens zwei unterschiedliche Ausgangsstellungen (32; 33) aufweist, ausgehend von denen die Betätigungsvorrichtung (3) zur Ausschüttung des Produktes in Bewegung gesetzt werden kann;
e) eine Rückstellvorrichtung (10), die die Betätigungsvorrichtung (3) nach der Bewegung aus der einen Ausgangsstellung (32; 33) heraus zurück bewegt in die andere Ausgangsstellung (33; 32);
und wobei das Verabreichungsgerät so ausgebildet ist, dass der Bewegung der Betätigungsvorrichtung (3) aus den unterschiedlichen Ausgangsstellungen (32; 33) heraus unterschiedliche Mengen an auszuschüttendem Produkt zugeordnet sind.

2. Verabreichungsgerät gemäß dem vorhergehenden Anspruch, wobei die eine Menge des ausgeschütteten fluiden Produktes die Primingmenge und die andere Menge des ausgeschütteten fluiden Produktes die Wirkstoffmenge ist, die zum dosierten Wirkstoffeinsatz benutzt wird.

3. Verabreichungsgerät gemäß einem der vorhergehenden Ansprüche, wobei das Betätigen der Betätigungsvorrichtung aus einer Bewegung eines Elementes der Betätigungsvorrichtung entlang der Längsachse des Verabreichungsgerätes besteht.

4. Verabreichungsgerät gemäß einem der vorhergehenden Ansprüche, bei dem die Rückstellvorrichtung (10) ein elastisches Element umfasst.

5. Verabreichungsgerät gemäß einem der vorhergehenden Ansprüche, wobei eine Rückwärtsbewegung der Betätigungsvorrichtung (3) in zwei verschiedenen Ausgangsstellungen (32;33) endet.

6. Verabreichungsgerät gemäß einem der vorhergehenden Ansprüche, wobei die Betätigungsvorrichtung (3) eine Endstellung (41) aufweist, durch die die Bewegung der Betätigungsvorrichtung (3) zur Beendigung der Ausschüttung des fluiden Produktes begrenzt wird.

7. Verabreichungsgerät gemäß dem vorhergehenden Anspruch, wobei die Endstellung (41) durch einen Anschlag definiert wird.

8. Verabreichungsgerät gemäß einem der vorhergehenden Ansprüche mit einem Kopplungsstück (25), das die Betätigungsvorrichtung (3) mit der Antriebsvorrichtung (11) koppelt.

9. Verabreichungsgerät gemäß dem vorhergehenden Anspruch mit zwei Anschlägen (32; 33), die mit dem Kopplungsstück (25) wechselweise in Verbindung gebracht werden.

10. Verabreichungsgerät gemäß dem vorhergehenden Anspruch, wobei einer der Anschläge (32; 33) ein Priminganschlag (33) und der andere ein Wirkstoffanschlag (32) ist.

11. Verabreichungsgerät gemäß einem der Ansprüche 8 bis 10, wobei das Kopplungsstück (25) aus einem Übertragungsstück (30) und einem Schiebestück (20) besteht.

12. Verabreichungsgerät gemäß dem vorhergehenden Anspruch, wobei das Übertragungsstück (30) zweischenklig (31a; 31b) ausgebildet ist.

13. Verabreichungsgerät gemäß einem der Ansprüche 9 bis 10 und gemäß einem der Ansprüche 11 bis 12, wobei das Übertragungsstück (30) zwei gewinkelte Abschnitte (34a; 34b) aufweist, die mit den zwei Anschlägen (32; 33) verhaken.

14. Verabreichungsgerät gemäß dem vorhergehenden Anspruch, wobei das Übertragungsstück (30) kippbar und/oder drehbar um die Anschläge (32; 33) ist.

15. Verabreichungsgerät gemäß einem der Ansprüche 11 bis 14, wobei das Schiebestück (20) in einer Richtung orthogonal zur Längsachse (L) des Verabreichungsgerätes verschoben wird.

16. Verabreichungsgerät gemäß einem der vorhergehenden Ansprüche, wobei die Anschläge (32; 33) als Rastanschläge ausgebildet sind.

17. Verabreichungsgerät gemäß einem der vorhergehenden Ansprüche, das ein Dosierglied aufweist, mit dem die zu verabreichende Wirkstoffmenge des fluiden Produktes eingestellt wird.

18. Verabreichungsgerät gemäß einem der vorhergehenden Ansprüche, wobei die Abtriebsvorrichtung (8) einen Kolben aufweist, der bei Produktverabreichung in eine Richtung entlang der Längsachse (L) des Verabreichungsgerätes bewegt wird und der an einer Bewegung in die entgegengesetzte Richtung durch eine Sperrvorrichtung (9) gehindert wird.

19. Verfahren zur Verwendung des Verabreichungsgerätes gemäß einem der vorhergehenden Ansprüche, das die folgenden Schritte umfasst:
a) erstes Betätigen der Betätigungsvorrichtung (3;40) in einer ersten Ausgangsstellung (33);
b) Ausschütten einer ersten Menge eines fluiden Produktes;
c) Rückstellen der Betätigungsvorrichtung (3;40) in eine zweite Ausgangsstellung (32);
d) zweites Betätigen der Betätigungsvorrichtung (3;40) in der zweiten Ausgangsstellung (32);
e) Ausschütten einer zweiten Menge eines fluiden Produktes;
f) Rückstellen der Betätigungsvorrichtung (3;40) in die erste Ausgangsstellung (33).

20. Verfahren zur Verwendung des Verabreichungsgerätes gemäß dem vorhergehenden Anspruch, wobei die Schritte des Verfahrens zyklisch wiederholt werden.

## Claims

1. Device for delivering/administering a fluid product, comprising:
a) A housing (7) with a reservoir (6) for the product;
b) A driven device (8), which is formed in such a way as to discharge the product, when it is in the reservoir;
c) A drive device (11), acting on the driven device (8);
d) An actuating device (3),
- which can be moved.
- which is coupled with the drive device (11), and
- which has at least two different output positions (32; 33), with the assistance of which the actuating device (3) can be set in motion for the discharge of the product;
e) A reset device (10), which returns the actuating device (3) after the movement from one of the output positions (32; 33) to the other output position (33; 32); and in which the delivery/administering device is formed in such a way, that the movements of the actuating device (3) from the different output positions (32; 33) are assigned to different quantities of the product to be discharged.

2. Device for delivering a fluid product according to the foregoing claim, in which one quantity of the discharged fluid product is the priming quantity and the other quantity is the discharged fluid product constituting the actual dose.

3. Device for delivering a fluid product according to one of the foregoing claims, in which the actuation of the actuating device consists of a movement of an element of the actuating device along the longitudinal axis of the delivery device.

4. Device for delivering a fluid product according to one of the foregoing claims, in which the reset device (10) comprises an elastic element.

5. Device for delivering a fluid product according to one of the foregoing claims, in which a backward movement of the actuating device (3) ends in two different output positions (32; 33).

6. Device for delivering a fluid product according to one of the foregoing claims, in which the actuating device (3) has an end position (41), at which the movement of the actuating device (3) is limited to terminate the discharge of the fluid product.

7. Device for delivering a fluid product according to one of the foregoing claims, in which the end position (41) is defined by a stop.

8. Device for delivering a fluid product according to one of the foregoing claims with a coupling element (25), which couples the actuating device (3) with the drive device (11).

9. Device for delivering a fluid product according to the foregoing claim with two stops (32; 33), which are alternately brought into connection with the coupling element (25).

10. Device for delivering a fluid product according to the foregoing claim, in which one of the stops (32; 33) is a priming stop (33) and the other is an ingredient stop (32).

11. Device for delivering a fluid product according to one of the claims 8 to 10, in which the coupling element (25) consists of a transfer element (30) and a sliding element (20).

12. Device for delivering a fluid product according to the foregoing claim, in which the transfer element (30) is formed with two arms (31a; 31b).

13. Device for delivering a fluid product according to one of the claims 9 and 10 and according to one of the claims 11 and 12, in which the transfer element (30) has two bent portions (34a; 34b), which engage with the two stops (32; 33).

14. Device for delivering a fluid product according to the foregoing claim, in which the transfer element (30) can be tilted and/or rotated around the stops (32; 33).

15. Device for delivering a fluid product according to one of the claims 11 to 14, in which the sliding element (20) is moved in a direction orthogonal to the longitudinal axis (L) of the delivery device.

16. Device for delivering a fluid product according to one of the foregoing claims, in which the stops (32; 33) are formed as latching stops.

17. Device for delivering a fluid product according to one of the foregoing claims, which has a metering element, with which the delivered/administered dose of the fluid product is adjusted.

18. Device for delivering a fluid product according to one of the foregoing claims, in which the driven device (8) has a piston, which is moved in a direction along the longitudinal axis (L) of the delivery device when the product is delivered and is prevented from movement in the opposite direction by a blocking device (9).

19. Method for the use of the fluid delivery device according to one of the foregoing claims, which comprises the following steps:
a) First actuation of the actuating device (3; 40) to a first output position (33);
b) Discharge of a first amount of a fluid product;
c) Reset of the actuating device (3; 40) to a second output position (32);
d) Second actuation of the actuating device (3; 40) to the second output position (32);
e) Discharge of a second amount of a fluid product;
f) Reset of the actuating device (3; 40) to the first output position (33).

20. Method for the use of the fluid delivery device according to the foregoing claim, in which the steps of the method are repeated as a cycle.

## Revendications

1. Dispositif d'administration destiné à l'administration d'un produit fluide, comprenant :
a) un boîtier (7) muni d'un réservoir (6) pour le produit ;
b) un dispositif d'expulsion (8) conçu pour déverser le produit lorsqu'il se trouve dans le réservoir ;
c) un dispositif d'entraînement (11) agissant sur le dispositif d'expulsion (8) ;
d) un dispositif d'actionnement (3),
- qui est mobile,
- qui est couplé avec le dispositif d'entraînement (11), et
- qui comporte au moins deux positions différentes de départ (32 ; 33), à partir desquelles le dispositif d'actionnement (3) peut être mis en mouvement pour déverser le produit ;
e) un dispositif de repositionnement (10) qui, après le mouvement, ramène le dispositif d'actionnement (3) depuis une des positions de départ (32 ; 33) dans l'autre position de départ (33 ; 32) ;
et où le dispositif d'administration étant conçu de telle sorte que différentes quantités de produit à déverser sont attribuées au mouvement du dispositif d'actionnement (3) depuis les différentes positions de départ (32 ; 33).

2. Dispositif d'administration selon la revendication précédente, où la quantité de produit fluide déversé correspond à la quantité d'amorçage et l'autre quantité de produit fluide déversé correspondant à la quantité de substance active qui est utilisée pour l'emploi dosé de substance active.

3. Dispositif d'administration selon l'une quelconque des revendications précédentes, où l'actionnement du dispositif d'actionnement se compose d'un mouvement d'un élément du dispositif d'actionnement le long de l'axe longitudinal du dispositif d'administration.

4. Dispositif d'administration selon l'une quelconque des revendications précédentes, dans lequel le dispositif de repositionnement (10) comprend un élément élastique.

5. Dispositif d'administration selon l'une quelconque des revendications précédentes, où un mouvement rétrograde du dispositif d'actionnement (3) se termine par deux positions différentes de départ (32 ; 33).

6. Dispositif d'administration selon l'une quelconque des revendications précédentes, où le dispositif d'actionnement (3) comporte une position de fin de course (41) par laquelle le mouvement du dispositif d'actionnement (3) est limité pour achever le déversement du produit fluide.

7. Dispositif d'administration selon l'une quelconque des revendications précédentes, où la position de fin de course (41) est définie par une butée.

8. Dispositif d'administration selon l'une quelconque des revendications précédentes avec un élément de couplage (25) qui couple le dispositif d'actionnement (3) avec le dispositif d'entraînement (11).

9. Dispositif d'administration selon la revendication précédente avec deux butées (32 ; 33) qui sont mises tour à tour en liaison avec l'élément de couplage (25).

10. Dispositif d'administration selon la revendication précédente, où une des butées (32 ; 33) est une butée d'amorçage (33) et l'autre une butée de substance active (32).

11. Dispositif d'administration selon l'une quelconque des revendications 8 à 10, où l'élément de couplage (25) se compose d'un élément de transmission (30) et d'un élément coulissant (20).

12. Dispositif d'administration selon la revendication précédente, où l'élément de transmission (30) est configuré avec deux branches (31a ; 31b).

13. Dispositif d'administration selon l'une quelconque des revendications 9 à 10 et selon l'une quelconque des revendications 11 à 12, où l'élément de transmission (30) comporte deux sections coudées (34a ; 34b) qui accrochent les deux butées (32 ; 33).

14. Dispositif d'administration selon la revendication précédente, où l'élément de transmission (30) est basculant et/ou rotatif autour des butées (32 ; 33).

15. Dispositif d'administration selon l'une des revendications 11 à 14, où l'élément coulissant (20) est déplacé dans un sens orthogonalement à l'axe longitudinal (L) du dispositif d'administration.

16. Dispositif d'administration selon l'une quelconque des revendications précédentes, où les butées (32 ; 33) sont conçues comme des butées d'encliquetage.

17. Dispositif d'administration selon l'une quelconque des revendications précédentes, lequel comporte un organe de dosage avec lequel la quantité de substance active à administrer du produit fluide est réglée.

18. Dispositif d'administration selon l'une quelconque des revendications précédentes, où le dispositif d'expulsion (8) comporte un piston qui, lors de l'administration du produit, est déplacé dans un sens le long de l'axe longitudinal (L) du dispositif d'administration et qu'un dispositif de blocage (9) empêche de se déplacer en sens inverse.

19. Procédé d'utilisation du dispositif d'administration selon l'une quelconque des revendications précédentes, lequel comprend les étapes suivantes :
a) premier actionnement du dispositif d'actionnement (3 ; 40) dans une première position de départ (33) ;
b) déversement d'une première quantité d'un produit fluide ;
c) repositionnement du dispositif d'actionnement (3 ; 40) dans une seconde position de départ (32) ;
d) second actionnement du dispositif d'actionnement (3 ; 40) dans la seconde position de départ (32) ;
e) déversement d'une seconde quantité d'un produit fluide ;
f) repositionnement du dispositif d'actionnement (3 ; 40) dans la première position de départ.

20. Procédé d'utilisation du dispositif d'administration selon la revendication précédente, où les étapes du procédé sont réitérées de façon cyclique.
